(19) Europäisches Patentamt
European Patent Office
Office européen des brevets

(11) EP 1 734 036 B1

(12) **EUROPEAN PATENT SPECIFICATION**

(45) Date of publication and mention of the grant of the patent:
**31.08.2011 Bulletin 2011/35**

(21) Application number: **05012740.6**

(22) Date of filing: **14.06.2005**

(51) Int Cl.:
*C07C 303/40* (2006.01) *C07C 311/37* (2006.01)
*C07C 231/02* (2006.01) *C07C 231/12* (2006.01)
*C07C 233/51* (2006.01) *C07C 233/22* (2006.01)
*C07C 303/28* (2006.01) *C07C 309/73* (2006.01)

(54) **Process for preparation of tamsulosin and its derivatives**

Verfahren zur Herstellung von Tamsulosin und seinen Derivaten

Procédé pour la préparation de tamsulosine et ses dérivés

(84) Designated Contracting States:
**AT BE BG CH CY CZ DE DK EE ES FI FR GB GR HU IE IS IT LI LT LU MC NL PL PT RO SE SI SK TR**

(43) Date of publication of application:
**20.12.2006 Bulletin 2006/51**

(73) Proprietors:
- **Well-being Biochemical Corp.**
  **Taipei 114**
  **Taiwan, R.O.C. (TW)**
- **Taiwan Biotech Co., Ltd.**
  **Taoyuan City**
  **Tao Yuan Hsien (TW)**

(72) Inventors:
- **Jih, Ru Hwu**
  **Hsinchu City 300 (TW)**
- **Magendran, Balaachary**
  **Hsinchu City 300 (TW)**
- **Chakraborty, Subhasish K.**
  **Hsinchu City 300 (TW)**
- **Das, Asish R.**
  **Hsinchu City 300 (TW)**
- **Tsay, Shwu Chen**
  **Neihu District**
  **Taipei City 114 (TW)**
- **Sheu, Kuen Wang**
  **Taoyuan City**
  **Taoyuan County 330 (TW)**
- **Shu, Chun Mei**
  **Taoyuan City**
  **Taoyuan County 330 (TW)**
- **Lu, Chin Kun**
  **Taoyuan City**
  **Taoyuan County 330 (TW)**
- **Chang, Wei Min**
  **Taoyuan City**
  **Taoyuan County 330 (TW)**

(74) Representative: **Casalonga, Axel et al**
**Casalonga & Partners**
**Bayerstrasse 73**
**80335 München (DE)**

(56) References cited:
**WO-A-2004/087623** **WO-A-2004/101560**
**WO-A-2005/058810**

- **W.J. WHEELER, ET AL: "The synthesis of the 14C and 2H-isotopomers of (R)-N-[2-(2'-ethoxyphenoxy)-ethyl]-N-2-[3- (4'-methoxy-3'-sulphonamido)phenyl]propyl- amine hydrochloride, an alpha1-adrenoreceptor antagonist" JOURNAL OF LABELLED COMPOUNDS AND RADIOPHARMACEUTICALS, vol. 27, no. 2, 1989, pages 171-180, XP008049771 SUSSEX, GB ISSN: 0362-4803**
- **T.H. APPLEWHITE, ET AL.: "The interaction of alpha-chymotrypsin with a series of alpha-N-acetyl-alpha-amino acid methylamides" JOURNAL OF THE AMERICAN CHEMICAL SOCIETY., vol. 81, no. 9, 5 May 1959 (1959-05-05), pages 2208-2213, XP002367468 AMERICAN CHEMICAL SOCIETY, WASHINGTON, DC, US**
- **A. BASAK, ET AL.: "Chemoselective O-methylation of phenols under non-aqueous condition" TETRAHEDRON LETTERS., vol. 39, no. 27, 2 July 1998 (1998-07-02), pages 4883-4886, XP004120784 ELSEVIER SCIENCE PUBLISHERS, AMSTERDAM, NL ISSN: 0040-4039**

EP 1 734 036 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- L. BERLINGUET: "A new general synthesis of 2-amino alcohols" CANADIAN JOURNAL OF CHEMISTRY, vol. 32, 1954, pages 31-39, XP008059937 NATIONAL RESEARCH COUNCIL, OTTAWA, CA
- F. CAMPOS, ET AL.: "An efficient enantioselective synthesis of (R,R)-formoterol, a potent bronchodilator, using lipases" TETRAHEDRON: ASYMMETRY, vol. 11, no. 13, 14 July 2000 (2000-07-14), pages 2705-2717, XP004228722 ELSEVIER SCIENCE PUBLISHERS, AMSTERDAM, nl ISSN: 0957-4166
- T.R. ELWORTHY, ET AL.: "N-Arylpiperazinyl-N-propylamino deroivatives of heteroaryl amides as functional uroselective alpha1-adrenoreceptor antagonists" JOURNAL OF MEDICINAL CHEMISTRY., vol. 40, no. 17, 15 August 1997 (1997-08-15), pages 2674-2687, XP002191535 AMERICAN CHEMICAL SOCIETY, WASHINGTON, DC, US ISSN: 0022-2623

## Description

The present invention related to a new process for the preparation of the compounds of the formula 1 (where $R^1$ and $R^2$ represent $C_1$-$C_4$ alkyl group) and their hydrochloride thereof, and other various pharmaceutical used salts. The process is more efficient than the reported processes in total yield and reaction steps for the synthesis of compounds having the formula **1**. In the present invention, the use of optically pure starting material can provide the desired compound in more specific range to meet the pharmaceutical requirement. Furthermore, the shorter reaction steps will provide the desired drugs with limit scope of impurity profile.

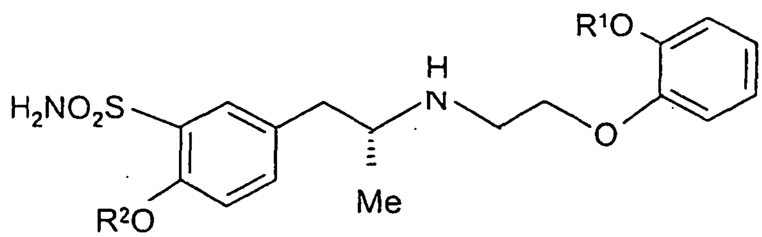

**1**

It is described in the U.S. Patent No. 5,447,958 that the compounds of the above formula 1 have excellent therapeutic effects against hypertension, congestive heart failure, angina pectoris or prostatic hypertrophy. In addition, the above patent disclosed a process for the preparation of compounds with the formula 1 by reacting following hydrochlorides of sulfonamide **2A** with bromide 3 :

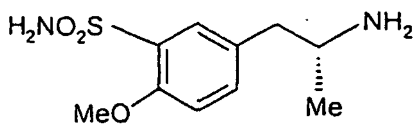

**2A**

+

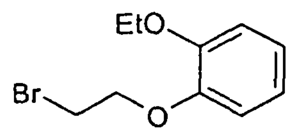

**3**

Wherein, sulfonamide hydrochloride salt **2B** (hydrochloride of formula **2A**) can be synthesized from the Process 1 depicted below. The synthetic procedure of Process 1 involves phenylamine **4A** as the starting material to produce the intermediates of acetamide **5A** and (sulfo)acetamide **6A,** and then to give sulfonamide hydrochloride salt **2B.**

Process 1 :

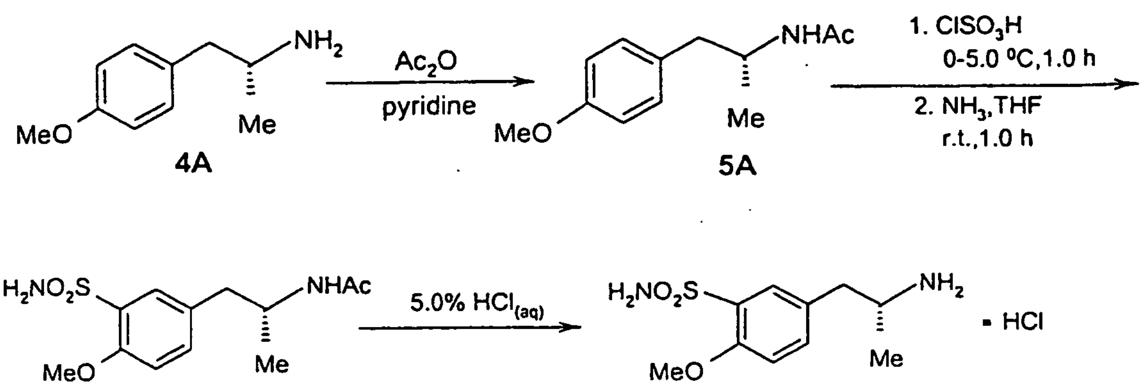


The synthesis of phenylamine **4A** was disclosed in U.S. Patent No. 4,000,197 and J. Med. Chem. 1973, 16, 480-483 by condensation of 4-methoxyphenylacetone with (*R*)-α-methylbenzylamine, followed by hydrogenation of the

N=N double bond therein, and reductive debenzylation. The total yield for the preparation of phenylamine **4A** is 25% with >99% enantiomeric purity.

The synthesis of tamsulosin by reaction of the hydrochloride of (R)-5-(2-aminopropyl)-2-methoxy-benzenesulfonamide with 2-(2-ethoxyphenoxy)ethylmesylate and tosylate is disclosed in WO 2004/087623.

Yamada et al. described a process for the synthesis of phenylamine **4A** in Synth. Commun. 1998, 28, 1935-1945 by using optically pure L-tyrosine as the starting material. The advantage of the current process is that the resultant product is optically pure. The preparation of intermediate **4B** (hydrochloride of phenylamine 4A) involves overall 8 steps, and if extended to intermediate **5A,** would be 9 steps. Yamada's procedure is depicted in Process 2 as below:

Process 2 :

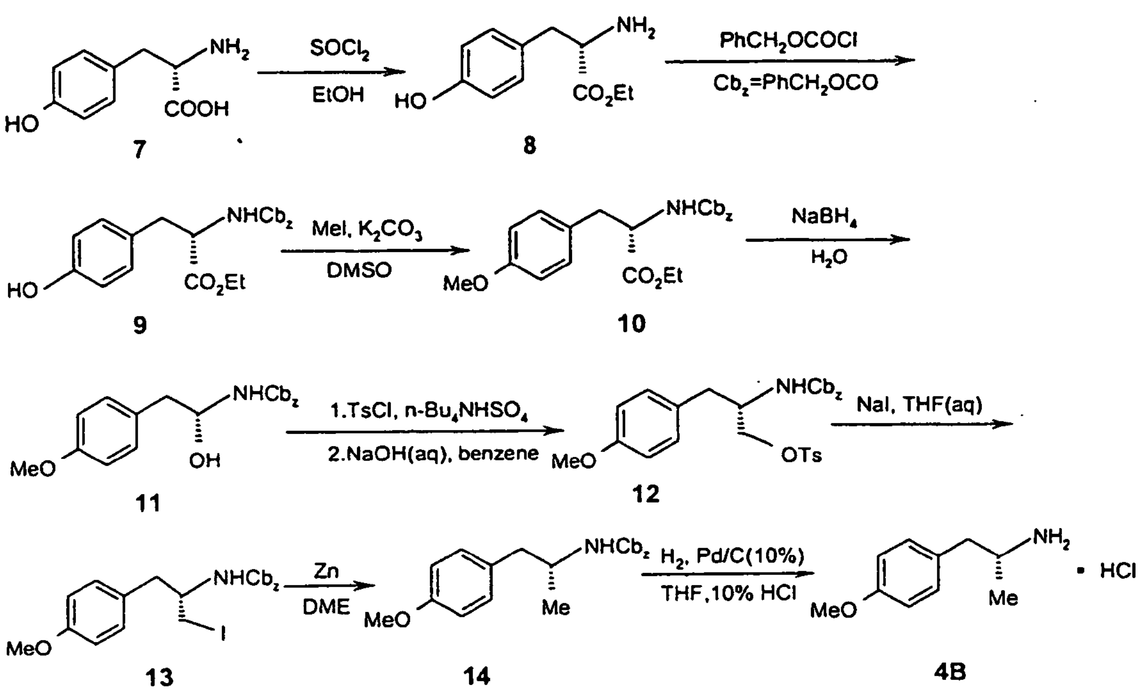


The synthetic procedure of Process 2 uses *L*-tyrosine **7** as the starting material to generate the intermediates of aminoester **8,** (phenol)amidoester **9,** (ether)amidoester **10,** (hydroxy)ether amide **11,** tosyl amide **12,** iodide **13,** ether amide **14,** and finally to obtain hydrochloride of phenylamine **4B.**

The present invention relates to a new process for the synthesis of sulfamoyl-substituted phenoethylamine derivatives and the acidic salts thereof, especially the tamsulosin derivatives having the following formula 1 (where $R^1$ and $R^2$ represent $C_1$-$C_4$ alkyl group) or their hydrochloride thereof, and other various pharmaceutical used salts.

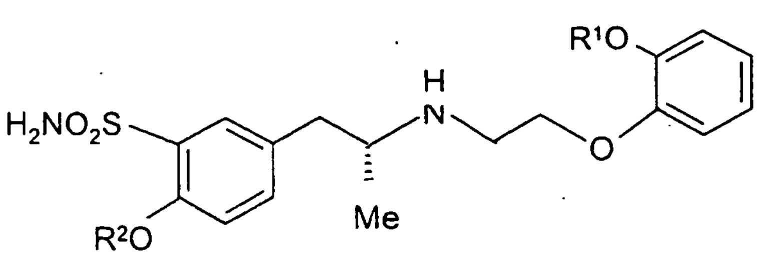

**1**

The process of the invention comprises:

reacting the hydrochloride of sulphonamide **2** with the ether compound **21** to obtain the tamsulosin **1**;

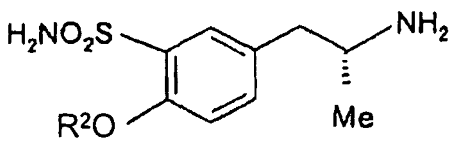

**2**

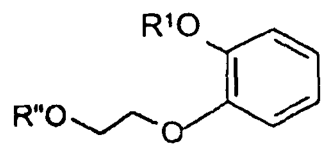

**21**

wherein in formulas **2** and **21**, $R^1$ and $R^2$ are as defined in formula **1**, and R" represent $MeC_6H_4SO_2$ or $MeSO_2$,

and involves the use, as key intermediate, of the following acetamide **5**:

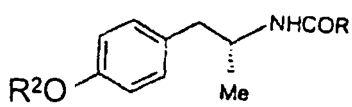

**5**

wherein R represents alkyl or aryl, and $R^2$ is as defined above,
wherein the intermediates as shown below are used for the preparation of acetamide **5**:

(1) the (phenol)amido acid 15 having the following formula:

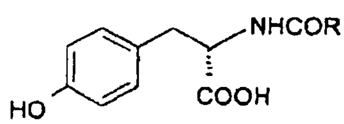

**15**

wherein R represents alkyl or aryl;
(2) the (phenol)amidoester 16 having the following formula:

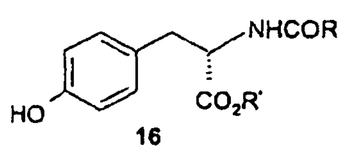

**16**

wherein R and R' represent alkyl or aryl;
(3) the (ether)amidoester 17 having the following formula:

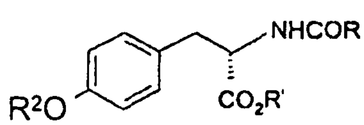

**17**

wherein R and R' represent alkyl or aryl; and $R^2$ is as defined above, and (4) the hydroxy(ether)amide 18 having the following formula:

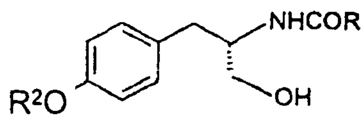

18

wherein R represents alkyl or aryl, and $R^2$ is as defined above.

As mentioned before, the synthesis of tamsulosin 1 ($R^1$ = Et and $R^2$ = Me) involves a key intermediate, i.e. acetamide **5A.** Herein, the invention discloses a new process, as shown in Process 3, for the preparation of the key intermediate **5A** as depicted below, where comprises the new intermediates, such as (phenol)amido acid **15A,** (phenol) amidoester **16A,** (ether)amidoester **17A,** hydroxy(ether)amide **18A.**

Process 3 :

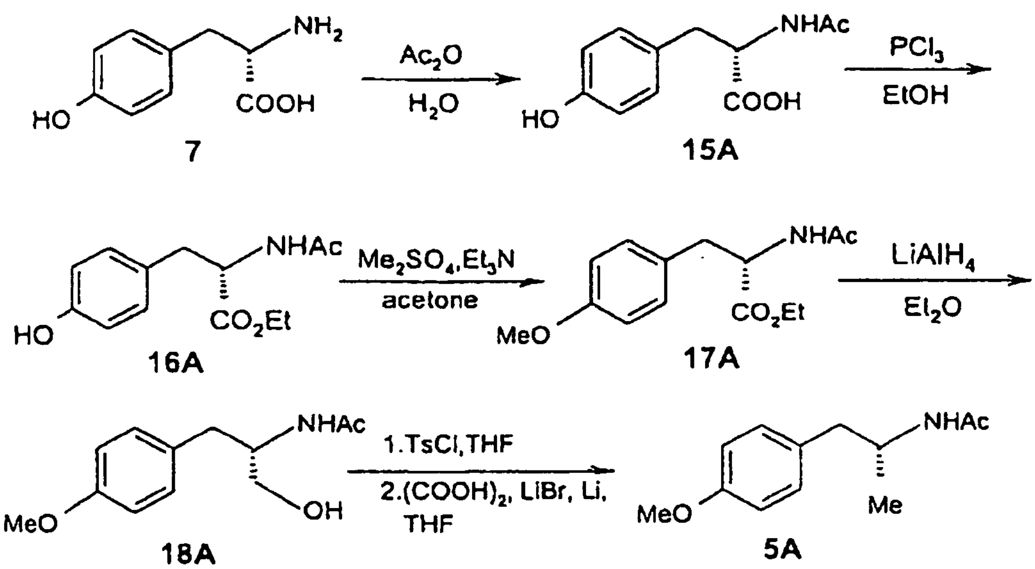


7, 15A, 16A, 17A, 18A, 5A

The process is more efficient than the reported processes in total yield and reaction steps for the synthesis of compounds having the formula 1. Furthermore, in the invention, the use of optically pure starting material can provide the desired compound in more specific range to meet the pharmaceutical requirement. And the shorter reaction steps will provide the desired drugs with limit scope of impurity profile.

According to a particular embodiment, a new key intermediate (ether)benzoxy tosylate **21A,** instead of bromide 3, for the preparation of formula 1 is also disclosed in the invention. It involves the use of 2-ethoxyphenol (**19A**) as the starting material. Sequential reaction of **19A** with chloroethanol and toluenesulfonyl chloride will provide the key intermediate **21A** via (ether)benzoxy alcohol **20A**. The advantages associated with the use of (ether)benzoxy tosylate **21A** instead of bromide 3 will simplify the manipulation of the reaction and, to a more important reason, avoid the pollution resulting from the hazardous halogenated wastes. The process for the preparation of (ether)benzoxy tosylate **21A** is depicted as shown in Process 4.

In the process of the invention, the compound **21** can be prepared by converting compound **19** hereunder to compound **20** hereunder, and then to compound **21**:

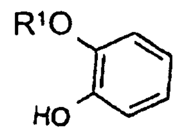

19

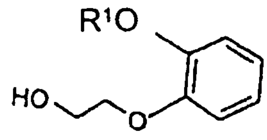

20

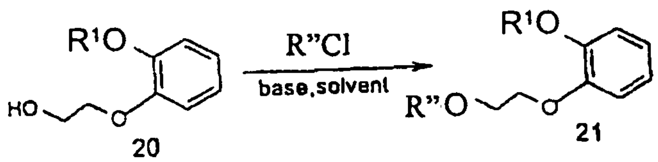


20

21

wherein a R"Cl, a base and a solvent are used; the R"Cl is selected from $MeC_6H_4SO_2Cl$ or $MeSO_2Cl$ and the combination thereof; the base is selected from $Et_3N$, $Me_3N$, pyridine and the combination thereof; the solvent is selected from $CH_2Cl_2$, acetone, THF, ether, ethyl acetate and the combination thereof.

Process 4 :

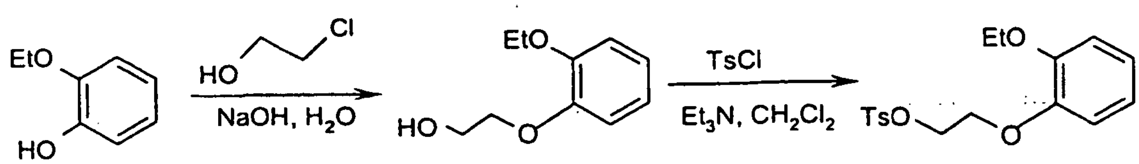


19A

20A

21A

The intermediate acetamide **5A** was converted to (sulfo)acetamide **6A** and then to sulfonamide hydrochloride salt **2B** by the established methods. Sulfonamide hydrochloride salt **2B** was allowed to react with (ether)benzoxy tosylate **21A** to generate the desired tamsulosin (1, $R^1$ = Et, $R^2$ = Me). The synthesis of tamsulosin is depicted as shown in Process 5.

Proeess 5 :

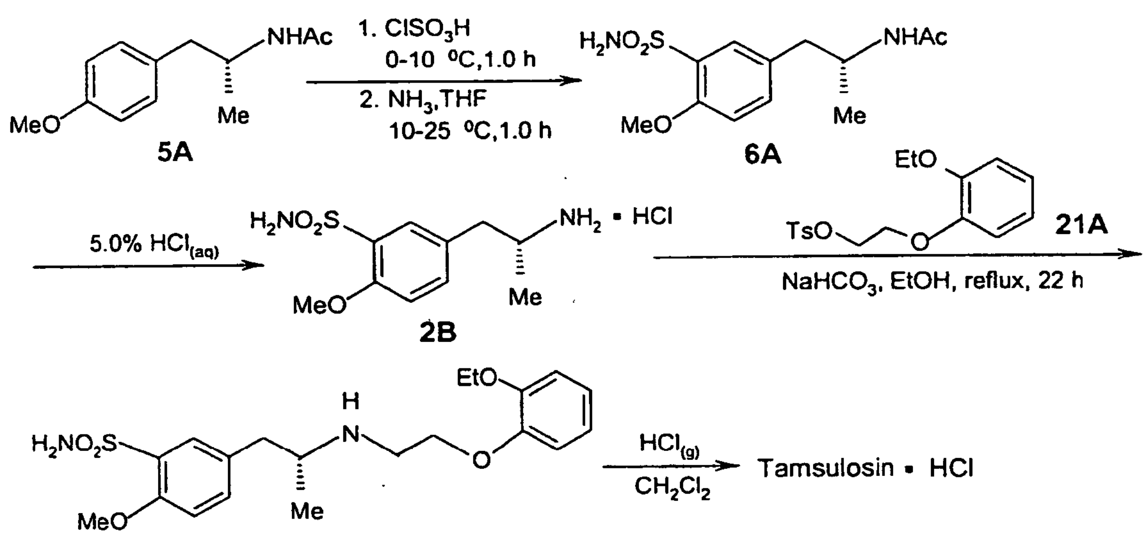


5A

6A

2B

21A

1

The present invention relates to a new process for the synthesis of sulfamoyl-substituted phenoethylamine derivatives and the acidic salts thereof.

A process for the preparation of (phenol)amido acid **15** from starting material L-tyrosine 7 is shown in Process 6.

Process 6 :

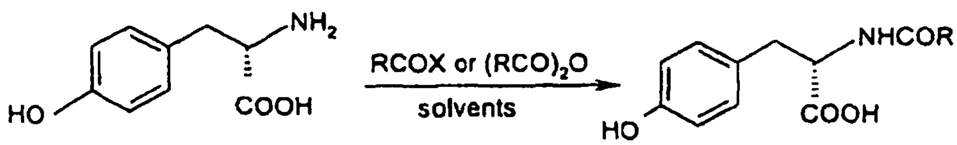


7 15

Additionally, an acylating agent and a solvent are used; the acylating agent is selected from RCOX, $(RCO)_2O$ and the combination thereof, wherein R is alkyl or aryl; X is a halide or a leaving group; the solvent is selected from alkanes, ethers, DMF, DMSO, ketones, urea and the combination thereof.

A process for the preparation of (phenol)amidoester **16** from (phenol)amido acid **15** is shown in Process 7.

Process 7 :

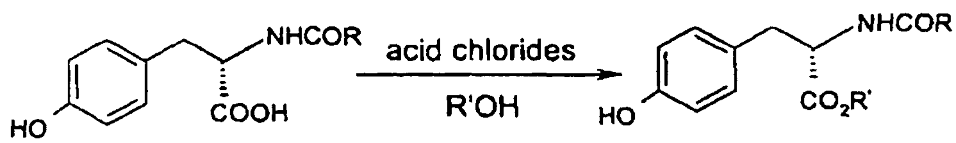


15 16

Additionally, an acid chloride and a R'OH are used; the acid chloride is selected from the group of $PCl_3$, $PCl_5$, $POCl_5$, $SOCl_2$, oxalyl chloride and the combination thereof; the R and R' groups are alkyl or aryl.

Furthermore, a process for the preparation of (ether)amidoester **17** from (phenol)amidoester **16** is shown in Process 8.

Process 8 :

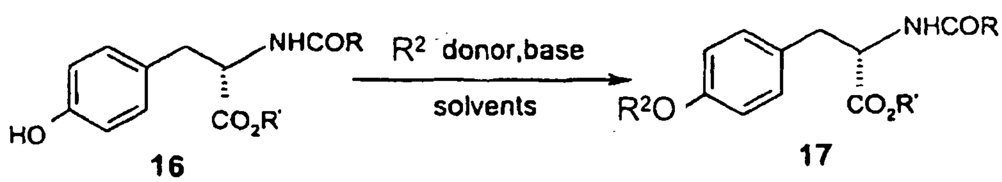


16 17

Additionally, an alkylating agent, a base and a solvent are used; the alkylating agent is selected from $R_{22}SO_4$, $R^2I$, $R^2Br$ and the combination thereof; the base is selected from amines, carbonates, hydrogen carbonates, amides, alkoxides and the combination thereof; the solvent is selected from $H_2O$, ketones, alkanes, ethers, DMF, DMSO, urea and the combination thereof.

A process for the preparation of hydroxy(ether)amide **18** from (ether)amidoester **17** is shown in Process 9.

Process 9 :

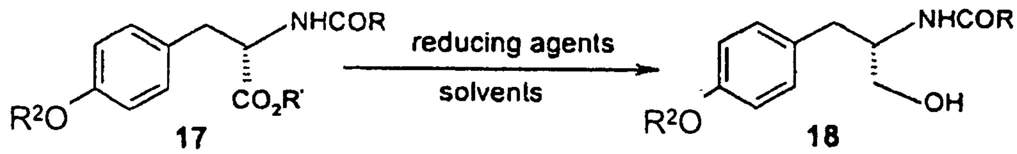


17 18

Additionally, a reducing agent and a solvent are used; the reducing agent is selected from $LiAlH_4$, DIBAL, K-selectride, L-selectride, $BH_3$, $NaBH_4$ and the combination thereof; the solvent is selected from ethers, alcohols, $H_2O$, alkanes, DMF, DMSO, urea and the combination thereof.

As shown in Process 10, acetamide **5** can be prepared from hydroxy(ether)amide **18**.

Process 10 :

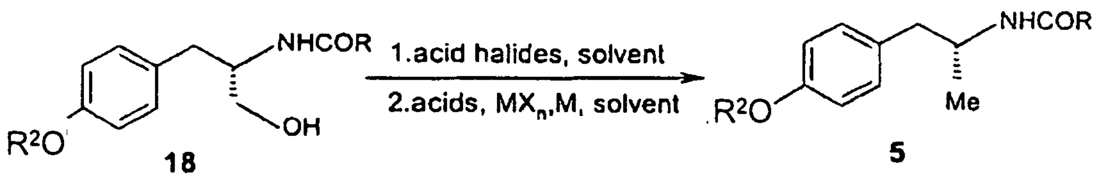


18 5

Wherein, an acid halide, a solvent, an organic acid, MXn and M are used; the acid halide is selected from the group of TsCl, MsCl, $SOCl_2$, $SO_2Cl_2$, $PCl_3$, $PCl_5$, $POCl_5$, oxalyl chloride and the combination thereof; the solvent is selected from THF, ketones, alkanes, ethers, DMF, DMSO, $CH_2Cl_2$, $CHCl_3$, $CCl_4$, urea and the combination thereof; the organic acid is selected from oxalic acid $(COOH)_2$, RCOOH and the combination thereof, where R is H, alkyl, or aryl; the M is selected from Li, Na, K, Mg, Ca, Zn, Pt, Pd, Cu, Co, Mn, Fe, Ni, or Cd; the X is Cl, Br, I, or OAc; the n value is 1-3 based on the valence of the metal.

As mentioned before, the synthesis of tamsulosin 1 involves a key intermediate acetamide 5. Herein, the invention could prepare tamsulosin 1 from acetamide 5.

The present invention can be further understood by the following examples, which are used to illustrate the present invention, but not to limit the scope thereof.

Example 1

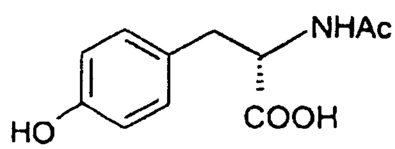

**15A**

To a solution of *L*-tyrosine **7** (20.01 g, 110.4 mmol) in $H_2O$ (120 mL) was added acetic anhydride (13.51 g, 132.4 mmol). After being heated at reflux for 4.0-5.0 h, the solution was concentrated by distillation to give a light yellow syrupy residue. The residue was dissolved in acetone (80 mL) and the unreacted *L*-tyrosine **7** was removed by filtration. The filtrate was concentrated under the reduced pressure and the residue was redissolved in ethyl acetate (100 mL), washed with water (50 mL), dried over $MgSO_4$ (s), and concentrated under reduced pressure to obtain (phenol)amido acid **15A** (17.62 g, 78.93 mmol) as gel-like semi-solid in 71% crude yield: mp (recrystallized from MeOH) 152-153 °C; specific rotation $[\alpha]_D^{20}$ = +50.9720°; $^1H$ NMR ($D_2O$, 400 MHz) δ 2.07 (s, 3 H, $COCH_3$), 2.82-2.86 (m, 1 H, ArC*H*H), 2.96-3.01 (m, 1 H, ArCH*H*), 3.67-3.70 (m, 1 H, CHCOO), 6.70 (d, J = 8.0 Hz, 2 H, ArH), 7.01 (d, J = 8.0 Hz, 2 H, ArH);

IR (neat) 3207 (s), 2961 (m), 1609 (s), 1591 (s), 1513 (s), 1455 (s), 1417 (s), 1363 (s), 1331 (s), 1267 (m), 1245 (s), 1214 (m), 1154 (m), 1112 (m), 1099 (m), 1042 (m), 984 (w), 939 (w), 897 (w), 877 (m), 841 (s), 794 (m), 740 (m), 713 (w), 649 (m), 575 (s), 529 (s), 493 (m), 433 (m) $cm^{-1}$.

Example 2

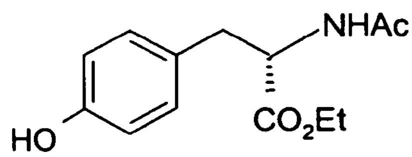

**16A**

To a solution of (phenol)amido acid **15A** (10.01 g, 44.84 mmol) in ethanol (300 mL) was added phosphorus trichloride (18.46 g, 134.4 mmol) dropwisely under ice-salt cooling (5 ˚C) over a period of 60 min. After being stirred at room temperature overnight, the reaction mixture was concentrated under reduced pressure. The residue was dissolved in ethyl acetate (50 mL), washed with water and saturated aqueous $NaHCO_3$ (25 mL), dried over $MgSO_4$ (s), and concentrated under reduced pressure to obtain (phenol)amidoester **16A** (8.225 g, 32.73 mmol) as pale yellow solids in 73% crude yield: mp (recrystallized from 50% EtOAc in hexanes) 126.0-128.0 ˚C; specific rotation $【\alpha】^{20}_{D}$ =-1.7632˚; $^1H$ NMR ($CDCl_3$, 400 MHz) δ 1.24 (t, J=6.8 Hz, 3 H, $CH_2CH_3$), 1.97 (s, 3 H, $COCH_3$) 2.95-3.08 (m, 2 H, $ArCH_2$), 4.16 (q, J=6.8 Hz, 2 H, $COCH_2$), 4.79-4.82 (m, 1 H, CHCOO), 6.71 (d, J = 8.0 Hz, 2 H, ArH), 6.94 (d, J = 8.0 Hz, 2 H, ArH); IR (neat) 3384 (br), 3020 (m), 2927 (m), 2851 (m), 1733 (s), 1652 (s), 1615 (s), 1542 (m), 1516 (s), 1446 (m), 1376 (m), 1219 (s), 1125 (w), 1026 (w), 828 (w), 769 (s), 668 (m), 518 (m) $cm^{-1}$.

Example 3

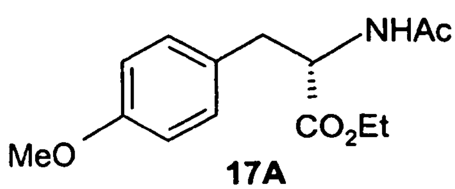

**17A**

To a solution of (phenol)amidoester **16A** (8.021 g, 31.92 mmol) in acetone (35 mL) was added triethylamine (7.365 g, 72.78 mmol) and dimethyl sulfate (5.473 g, 43.39 mmol). After being stirred at 25 ˚C for 20 h, the solution was quenched with water (50 mL) and extracted with toluene (50 mL). The organic layer was washed with 10% aqueous NaOH (25 mL) and brine (25 mL), dried over $MgSO_4$(s), filtered, and concentrated under reduced pressure to obtain (ether)amidoester **17A** (7.113 g, 26.81 mmol) as light brown semi-solids in 84% crude yield: mp (recrystallized from EtOAc) 140-142 ˚C; specific rotation $【\alpha】^{20}_{D}$ : = -1.6950˚; $^1H$ NMR ($CDCl_3$, 400 MHz) δ 1.21 (t, J = 6.8 Hz, 3 H, $CH_2CH_3$), 1.92 (s, 3 H, $COCH_3$), 3.02-3.09 (m, 2 H, $ArCH_2$), 3.95 (s, 3 H, $OCH_3$), 4.14 (q, J = 6.8 Hz, 2 H, $COCH_2$), 4.76-4.81 (m, 1 H, CHCOO), 6.76 (d, J = 8.0 Hz, 2 H, ArH), 7.02 (d, J = 8.0 Hz, 2 H, ArH); IR (neat) 3282 (m), 3075 (w), 2968 (m), 2933 (m), 2837 (w), 1716 (w), 1651 (s), 1614 (s), 1557 (s), 1514 (s), 1456 (s), 1374 (s), 1300 (m), 1248 (s), 1178 (m), 1146 (w), 1114 (w), 1035 (m), 975 (w), 815 (w), 775 (w), 608 (w), 562 (w), 523 (w), 419 (w) $cm^{-1}$.

Example 4

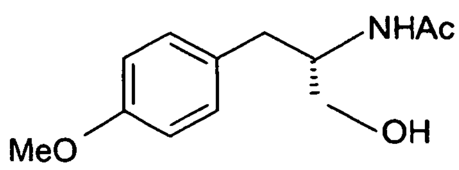

**18A**

To a stirred solution of $LiAlH_4$ (1.014 g, 26.72 mmol) in diethyl ether (140 mL) was added (ether)amidoester **17A** (7.088 g, 26.72 mmol) in diethyl ether (50 mL) under ice water cooling at 10 ˚C. After the solution was stirred at 25 ˚C for 10 h, the reaction mixture was neutralized with HCl (12 N, 10 mL) and filtered, and the filtrate was concentrated under reduced pressure. The residue was re-dissolved in ethyl acetate (200 mL), washed with water (20 mL), 10% aqueous NaOH (20 mL), brine (20 mL), dried over $MgSO_4$ (s), filtered, and concentrated under reduced pressure to obtain hydroxy(ether)amide **18A** (3.579 g, 16.03 mol) as white solids in 60% crude yield: mp (recrystallized from EtOAc and hexanes) 129-130 ˚C; specific rotation $[\alpha]_D^{20}$ = -10.9440˚; $^1$H NMR ($CDCl_3$, 400 MHz) δ 1.95 (s, 3 H, $NCOCH_3$), 2.40 (br, 1 H, OH), 2.75-2.85 (m, 2 H, $ArCH_2$), 3.57-3.67 (m, 2 H, $CH_2O$), 3.74 (s, 3 H, $OCH_3$), 4.08-4.14 (m, 1 H, CHN), 6.84 (d, J = 8.4 Hz, 2 H, ArH), 7.12 (d, J = 8.4 Hz, 2 H, ArH); IR (neat) 3512 (br), 3002 (w), 2948 (m), 2834 (m), 1892 (w), 1645 (s), 1577 (m), 1551 (m), 1511 (s), 1441 (m), 1377 (m), 1300 (s), 1245 (s), 1177 (s), 1083 (m), 1041 (s), 821 (m), 614 (m) $cm^{-1}$.

Example 5

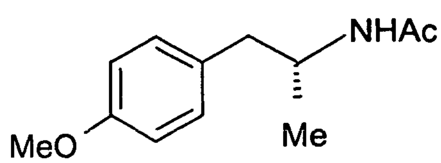

**5A**

To a solution ofhydroxy(ether)amide **18A** (10.01 g, 44.83 mmol) in THF (50 mL) was added toluenesulfonyl chloride (15.67 g, 82.19 mmol). After being stirred atreflux for 1.5 h, the reaction mixture was slowly poured into saturated aqueous $K_2CO_3$ solution. The aqueous layer was extracted with ethyl acetate (3 × 20 mL) and the combined organic layer was washed with water (80 mL), dried over $MgSO_4$ (s), and concentrated under reduced pressure. The residue was dissolved in THF (160 mL) containing oxalic acid (20 g) and LiBr (8.118 g, 93.47 mmol) was added. After being stirred at reflux for 10 min, the solution was added with Li (wire, 1.488 g, 214.4 mmol). The resultant solution was stirred at the same temperature for 2.0 h, filtered through celite, washed with ethyl acetate, and concentrated under reduced pressure. The residue was dissolved into water (50 mL), neutralized with $K_2CO_3$ (s, 20.01 g), and extracted with ethyl acetate (5 × 80 mL). The organic layer was washed with water (40 mL) and concentrated under reduced pressure. The residue was added with water (20 mL), kept for overnight, filtered, and dried under vacuum to obtain acetamide **5A** (6.711 g, 32.37 mmol) as white solids in 72% crude yield: mp (recrystallized from EtOAc) 90-91 ˚C; specific rotation $[\alpha]_D^{20}$ = + 9.9083˚; $^1$H NMR ($CDCl_3$, 300 MHz) δ 1.08 (d, J = 5.4 Hz, 3 H, $CCH_3$), 1.92 (s, 3 H, $COCH_3$), 2.60-2.77 (m, 2 H, $ArCH_2$), 3.76 (s, 3 H, $OCH_3$), 4.10-4.25 (m, 1 H, CHMe), 6.81 (d, J = 7.8 Hz, 2 H, ArH), 7.06 (d, J = 7.8 Hz, 2 H, ArH); IR (neat) 3276 (br), 3077 (m), 2969 (m), 2933 (m), 2836 (m), 1716 (m), 1647 (s), 1615 (s), 1542 (s), 1513 (s), 1456 (m), 1374 (m), 1300 (m), 1247 (s), 1178 (m), 1035 (m), 814 (m), 755 (w), 518 (w), 420(w)$cm^{-1}$.

Example 6

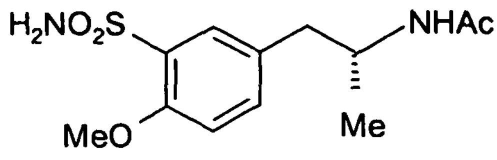

**6A**

To acetamide **5A** (1.01 g, 4.81 mmol) was added chlorosulfonic acid (10.1 g, 85.8 mmol) under cooling at 0-10 °C. The solution was stirred at 5.0 °C for 1.0 h. The reaction mixture was slowly poured into ice water and the resultant oily material was extracted with ethyl acetate (100 mL). The organic layer was washed with saturated aqueous $NaHCO_3$ (25 mL), $H_2O$ (10 mL), dried over $MgSO_4$(s), filtered, and concentrated under reduced pressure. The residue was redissolved in THF (20 mL), added with concentrated aqueous ammonia solution (15 N, 30 mL), and stirred at 25 °C for 1.0 h. The solution was concentrated under reduced pressure, and the resultant residue was washed with water (2.0 mL) and dried under reduced pressure to obtain (sulfo)acetamide **6A** (602.1 mg, 2.102 mmol) as white solids in 44% crude yield: mp (recrystallized from MeOH) 198-199 °C; specific rotation $[\alpha]_D^{20}$ = +13.2634°; $^1$H NMR ($D_2O$, 400 MHz) δ 0.99 (d, J = 6.8 Hz, 3 H, $CCH_3$), 1.68 (s, 3 H, $COCH_3$), 2.43-2.49 (m, 1 H, ArC*H*H), 2.69-2.74 (m, 1 H, ArCH*H*), 3.72 (s, 3 H, $OCH_3$), 3.80-3.86 (m, 1 H, CHMe), 7.01 (d, J = 8.8 Hz, 1 H, ArH), 7.33 (d, J = 8.8 Hz, 1 H, ArH), 7.48 (s, 1 H, ArH); IR (neat) 3132 (br), 1654 (s), 1609 (m), 1536 (m), 1496 (m), 1401 (s), 1320 (m), 1283 (m), 1253 (m), 1176 (w), 1148 (s), 1070 (m), 1024 (m), 977 (w), 927 (w), 860 (w), 838 (w), 828 (w), 761 (m), 701 (w), 669 (w), 614 (m), 599 (m), 572 (m), 535 (m), 505 (m), 450 (m) $cm^{-1}$; ESI-MS m/z 287.27 ($M + H^+$).

Example 7

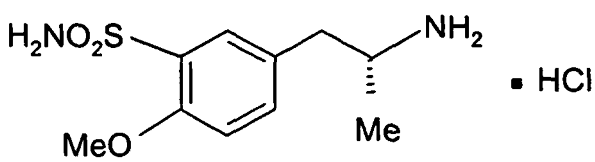

**2B**

An aqueous HCl solution (5.0%, 25 mL) containing (sulfo)acetamide **6A** (0.541 g, 1.88 mmol) was heated at reflux for 16 h. The solution was concentrated under reduced pressure, redissolved in hot MeOH (3.0 mL), and slowly added with ethyl acetate (10 mL). The precipitate was collected and dried under vacuum to obtain sulfonamide hydrochloride salt **2B** (0.449 g, 1.60 mmol) as white solids in 85% yield: mp (recrystallized from MeOH) 272-273 °C (dec.); specific rotation $[\alpha]_D^{20}$ = -9.2040°; $^1$H NMR ($D_2O$, 400 MHz) δ 1.14 (d, J = 6.4 Hz, 3 H, $CCH_3$), 2.79-2.82 (m, 2 H, $ArCH_2$), 3.43-3.56 (m, 1 H, CHMe), 3.84 (s, 3 H, $OCH_3$), 7.10 (d, J = 8.4 Hz, 1 H, ArH), 7.42 (d, J = 8.4 Hz, 1 H, ArH), 7.58 (s, 1 H, ArH); IR (neat) 3329 (s), 3196 (s), 3150 (s), 3025 (s), 2944 (s), 2701 (w), 2590 (w), 2501 (w), 1611 (m), 1553 (m), 1496 (s), 1402 (s), 1327 (s), 1282 (m), 1255 (m), 1154 (s), 1075 (m), 1017 (m), 928 (m), 828 (w), 804 (m), 703 (m), 601 (s), 572 (m), 536 (m), 507 (m) $cm^{-1}$; ESI-MS m/z 245.15 ($M + H^+$); Anal. Calcd for $C_{10}H_{17}N_2O_3SCl$: C, 42.78; H, 6.10; N, 9.98. Found: C, 42.76; H, 6.15; N, 9.93.

Example 8

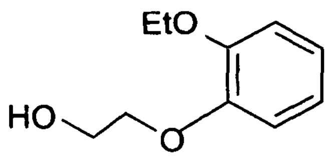

**20A**

To a solution of 2-ethoxyphenol **19A** (13.82 g, 100.1 mmol) in aqueous NaOH (1.0 N, 300 mL) was added chloroethanol (33.12 mL, 500.1 mmol). After being stirred at 25 ˚C for 48 h, the reaction mixture was extracted with ethyl acetate (3 x 100 mL). The organic layer was dried over $MgSO_4$(s), filtered, and concentrated under reduced pressure to obtain (ether)benzoxy alcohol **20A** (14.02 g, 76.94 mmol) as yellow liquid in 77 % yield: $^1$H NMR ($CDCl_3$, 400 MHz) δ 1.44 (t, J = 7.2 Hz, 3 H, $OCH_2CH_3$), 3.85 (t, J = 7.2 Hz, 2 H, $CH_2OH$), 4.05-4.12 (m, 4 H, $OCH_2CH_3$ + $OCH_2CH_2OH$), 6.88-6.97 (m, 4 H, ArH); IR (neat) 3547 (br), 3066 (m), 2977 (s), 2931 (s), 2877 (s), 1739 (m), 1649 (m), 1593 (s), 1503 (s), 1455 (s), 1393 (m), 1324 (m), 1253 (s), 1219 (s), 1123 (s), 1041 (s), 922 (s) $cm^{-1}$

Example 9

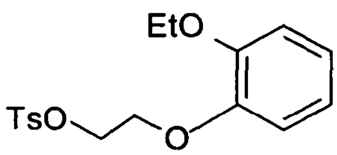

**21A**

To a solution of (ether)benzoxy alcohol **20A** (501.2 mg, 2.751 mmol) in $CH_2Cl_2$ (20 mL) was added $Et_3N$ (509 mg, 5.03 mmol) and toluenesulfonyl chloride (623 mg, 3.27 mmol) at 5-10 ˚C. After being stirred at 25 ˚C for 30 min, the reaction mixture was quenched with saturated aqueous $Na_2CO_3$ (20 mL) and extracted with ethyl acetate (3 × 50 mL). The combined organic layers were dried over $MgSO_4$(s), filtered, and concentrated under reduced pressure to obtain (ether)benzoxy tosylate 21A (853.5 mg, 2.539 mmol) as white solids in 92 % yield: mp (recrystallized from $CH_2Cl_2$ and $CCl_4$) 82-83 ˚C; $^1$H NMR ($CDCl_3$, 300 MHz) δ 1.43 (t, J = 10.2 Hz, 3 H, $OCH_2CH_3$), 2.51 (s, 3 H, $ArCH_3$), 4.04 (q, J = 10.2 Hz, 2 H, $OCH_2CH_3$), 4.36 (t, J = 6.9 Hz, 2 H, $OCH_2CH_2$), 4.59 (t, J = 6.9 Hz, 2 H, $OCH_2CH_2$), 6.86-6.97 (m, 4 H, ArH), 7.10-7.22 (m, 4 H, ArH); IR (neat) 3446 (br), 2982 (m), 2934 (m), 2884 (m), 1591 (s), 1558 (w), 1509 (s), 1478 (s), 1454 (s), 1407 (s), 1394 (s), 1371 (s), 1354 (s), 1279 (m), 1259 (s), 1247 (s), 1217 (s), 1178 (s), 1127 (s), 1066 (s), 1043 (s), 1031 (s), 977 (s), 929 (s), 905 (s), 809 (s), 776 (m), 749 (s), 776 (m) $cm^{-1}$

Example 10

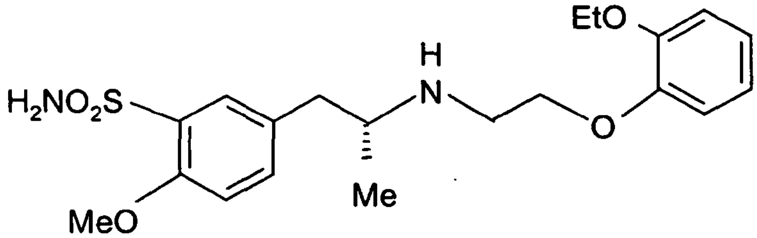

**1**

To a solution of sulfonamide hydrochloride salt **2B** (1.12 g, 4.00 mmol) in EtOH (40 mL) was added $NaHCO_3$ (672 mg, 8.00 mmol). After being stirred at room temperature for 5.0 min, the solution was added with (ether)benzoxy tosylate **21A** (1.34 g, 4.00 mmol). The solution was stirred at 90-100 °C for 22 h. The reaction mixture was cooled to room temperature, filtered, concentrated under reduced pressure, and dried under vacuum. The resultant semi-solid was dissolved in $CH_2Cl_2$ (50 mL), filtered, and the solid was washed with $CH_2Cl_2$ (3 × 5.0 mL). The residual solid was recovered as the unreacted sulfonamide **2A**. The filtrate was concentrated under reduced pressure, and the residue was dissolved in $CHCl_3$ (50 mL) and washed with water (3 × 25 mL). The aqueous solution was added with brine and then extracted with EtOAc to obtain the unreacted sulfonamide **2A.** The organic layer was dried over $MgSO_4$ (s), filtered, and concentrated under reduced pressure. The residue was dried under vacuum and the solid was washed with $CCl_4$ (3 × 20 mL). Ethoxyphenol **19A** was recovered by concentration of the $CCl_4$ solution. The solid was purified by column chromatography on silica gel (37% MeOH in $CHCl_3$ as eluant) to obtain tamsulosin **1** (571 mg, 1.40 mmol) as white solids in 35% yield: mp (recrystallized from $CH_2Cl_2$ and EtOAc) 129-131 °C; specific rotation $[\alpha]_D^{20}$ = -14.7240°; $^1$H NMR ($CD_3OD$, 400 MHz) δ 0.99 (d, J = 6.4 Hz, 3 H, $NCHCH_3$), 1.28 (t, J = 7.2 Hz, 3 H, $OCH_2CH_3$), 2.49-2.54 (m, 1 H, ArC*H*H), 2.77-2.82 (m, 1 H, ArCH*H*), 2.89-2.98 (m, 3H, $NCH_2$ + NCH), 3.88 (s, 3 H, $OCH_3$), 3.93-4.57 (m, 4 H, $CH_2CH_2O$ + $CH_3CH_2O$), 6.81-6.89 (m, 4 H, ArH), 7.03 (d, J = 8.4 Hz, 1 H, ArH), 7.36 (d, J = 8.4 Hz, 1 H, ArH), 7.63 (s, 1 H, ArH); IR (neat) 3284 (m), 2973 (m), 2939 (m), 1592 (m), 1504 (s), 1442 (m), 1324 (s), 1282 (m), 1249 (s), 1214 (m), 1154 (s), 1125 (m), 1073 (m), 1046 (m), 971 (w), 925 (m), 753 (m) $cm^{-1}$; ESI-MS m/z 409.40 (M + $H^+$).

Example 11

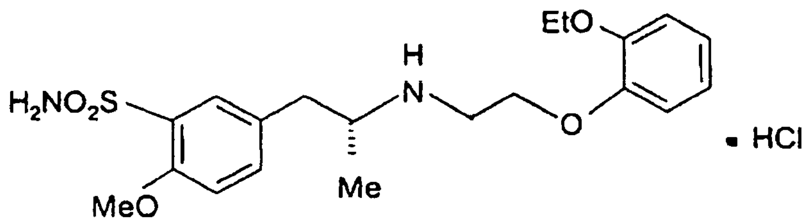

Tamsulosin · HCl

To a solution of tamsulosin **1** (2.011 g, 4.917 mmol) in $CH_2Cl_2$ (50 mL) was bubbled with excess dry $HCl_{(g)}$ at 0-5 °C for 1.0 h. The resultant precipitate was filtered and dried under vacuum at room temperature to obtain tamsulosin · HCl (2.091 g, 4.699 mmol) as white solids in 96% yield: mp (recrystallized from 50% MeOH in EtOH) 230-231 °C; specific rotation $[\alpha]_D^{20}$ = -5.3843°; $^1$H NMR ($D_2O$, 400 MHz) δ 1.14-1.17 (m, 6 H, $NCHCH_3$ + $OCH_2CH_3$), 2.71-2.76 (m, 1 H, ArC*H*H), 2.95-3.00 (m, 1 H, ArCH*H*), 3.36-3.43 (m, 2 H, $NCH_2$), 3.53-3.55 (m, 1 H, NCH), 3.75 (s, 3 H, $OCH_3$), 3.94-3.97 (m, 2 H, $MeCH_2O$), 4.04-4.19 (m, 2 H, $OCH_2CH_2$), 6.84-6.96 (m, 5 H, ArH), 7.35 (d, J = 8.8 Hz, 1 H, ArH), 7,54 (s, 1 H, ArH); IR (neat) 3304 (m), 3168 (m), 2981 (m), 1610 (m), 1589 (m), 1500 (s), 1458 (m), 1392 (m), 1339 (s), 1251 (s), 1215 (s), 1160 (s), 1128 (s), 1072 (m), 1046 (m), 1018 (m), 820 (m), 749 (s), 718(m)$cm^{-1}$.

In the present invention a process for preparation of tamsulosin and its aralkylamine derivatives is disclosed. The steps for preparation of tamsulosin from starting material L-tyrosine are fewer and the yield is higher than that of the conventional method, meanwhile, the key intermediates **15A** to **18A** as synthesized during tamsulosin 1 preparation are also representative intermediates for the present invention.

## Claims

**1.** A process for the preparation of tamsulosin and its derivatives, having the following formula:

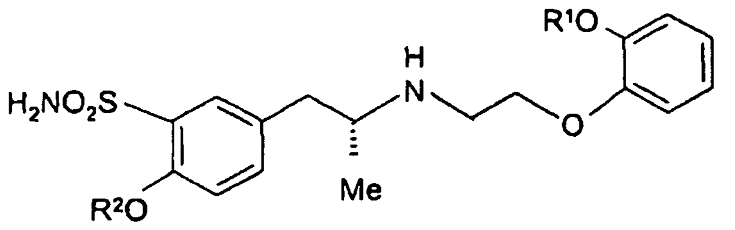

1

wherein $R^1$ and $R^2$ represent $C_1$-$C_4$ alkyl, or hydrochloride thereof, and other various pharmaceutical used salts, the process comprising :

reacting the hydrochloride of sulfonamide **2** with the ether compound **21** to obtain the tamsulosin **1**;

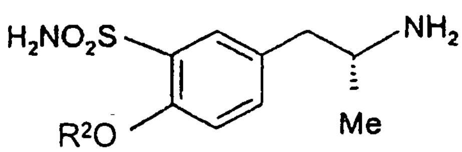

2

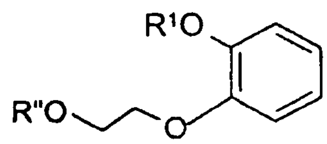

21

wherein in formula **2**, and 21, $R^1$ and $R^2$ are as defined in formula 1, and R" represents $MeC_6H_4SO_2$ or $MeSO_2$,
the process involving the use, as key intermediate, of the following acetamide 5 :

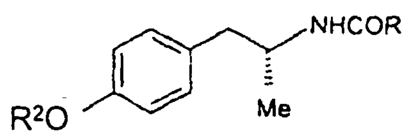

5

wherein R represents alkyl or aryl, and $R^2$ is as defined above, wherein the intermediates as shown below are used for the preparation of acetamide **5**:

(1) the (phenol)amido acid **15** having the following formula:

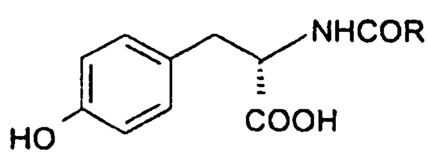

15

wherein R represents alkyl or aryl;
(2) the (phenol)amidoester **16** having the following formula:

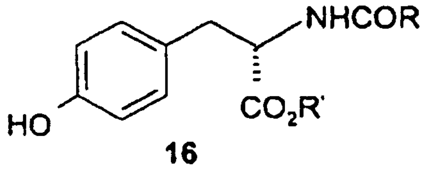

16

wherein R and R' represent alkyl or aryl;
(3) the (ether)amidoester **17** having the following formula:

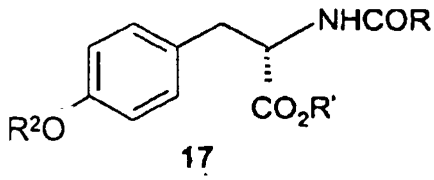

17

wherein R and R' represent alkyl or aryl; and $R^2$ is as defined above, and
(4) the hydroxy(ether)amide **18** having the following formula:

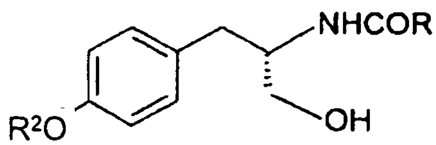

18

wherein R represents alkyl or aryl, and $R^2$ is as defined above.

**2.** The process for the preparation of tamsulosin as claimed in claim 1, when $R^1$ is ethyl and $R^2$ is methyl for tamsulosin 1, $R^2$ in formula **2** represents Me, as shown by **2A**, and the hydrochloride of **2A** is shown by **2B**; in ether compound **21**, $R^1$ represents Et and R" represents Ts, as shown by the (ether)benzoxy tosylate **21A**;

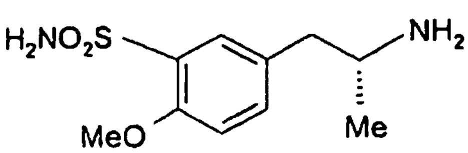

2A ;

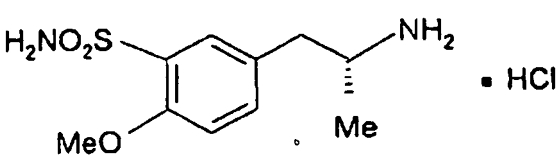

2B ;

and

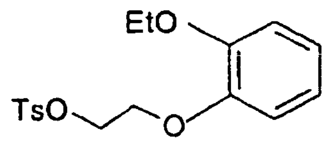

21A

3. The process for the preparation of tamsulosin as claimed in claim 2, wherein the preparation process for sulfonamide hydrochloride salt **2B** comprises :

converting acetamide **5A** to (sulfo)acetamide **6A** and then to obtain sulfonamide hydrochloride salt **2B** under an acidic condition, as shown by the below procedures:

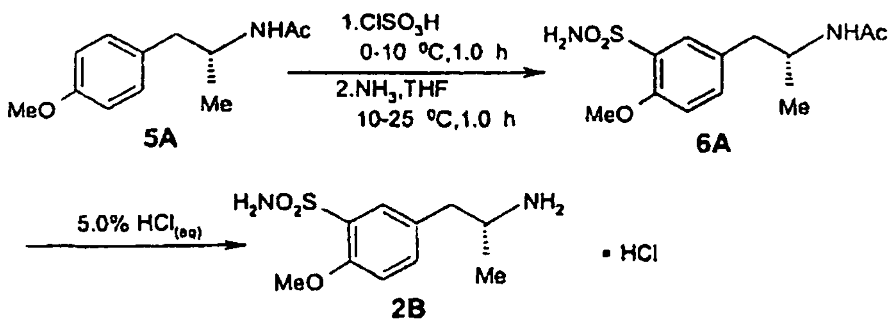


5A 6A

2B

4. The process for the preparation of tamsulosin as claimed in claim 1, wherein the compound **21** is prepared by converting compound 19 hereunder to compound 20 hereunder, and then to compound **21** :

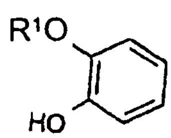

19

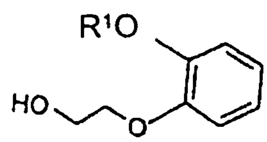

20

wherein $R^1$ is as defined in claim 1.

5. The process of claim 1, wherein the (phenol) amido acid **15** is prepared from starting material L-tyrosine 7, as follows :

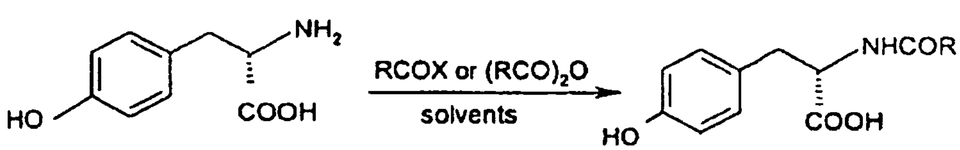


7 15

wherein an acylating agent and a solvent are used; the acylating agent is selected from RCOX, $(RCO)_2O$ and the combination thereof, wherein R is alkyl or aryl; X is a halide or a leaving group; the solvent is selected from alkanes, ethers, DMF, DMSO, ketones, urea and the combination thereof.

**6.** The process of claim 1, wherein the preparation of (phenol)amidoester 16 from (phenol)amido acid 15 comprises :

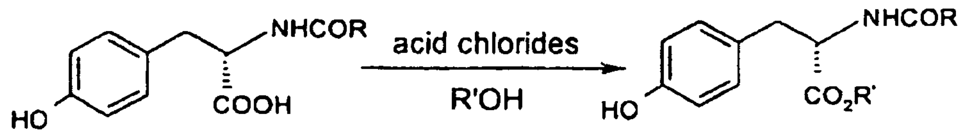


15 16

wherein an acid chloride and a R'OH are used; the acid chloride is selected from the group of $PCl_3$, $PCl_5$, $POCl_5$, $SOCl_2$, oxalyl chloride and the combination thereof;
the R and R' groups are alkyl or aryl.

**7.** The process of claim 1, wherein the preparation of (ether)amidoester **17** from (phenol)amidoester **16** comprises :

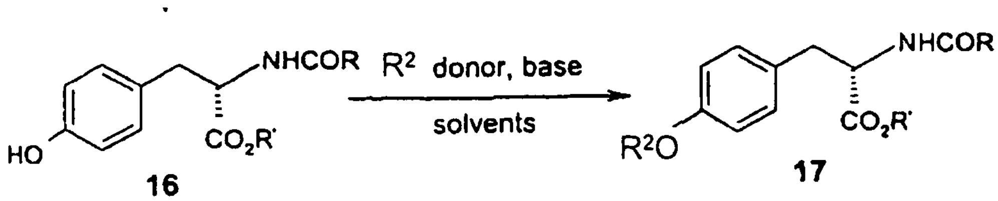


16 17

wherein an alkylating agent, a base and a solvent are used; the alkylating agent is selected from $R_2{}^2SO_4$, $R^2I$, $R^2Br$ and the combination thereof; the base is selected from amines, carbonates, hydrogen carbonates, amides, alkoxides and the combination thereof; the solvent is selected from $H_2O$, ketones, alkanes, ethers, DMF, DMSO, urea and the combination thereof.

**8.** The process of claim 1, wherein the preparation of hydroxy(ether)amide **18** from (ether) amidoester **17** comprises :

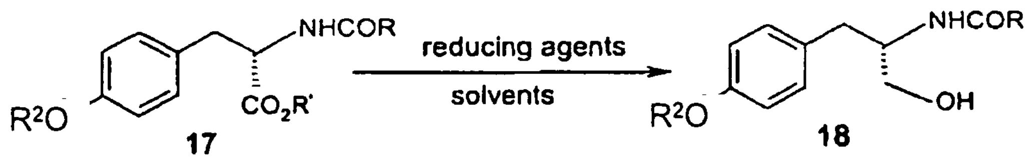


17 18

wherein a reducing agent and a solvent are used; the reducing agent is selected from $LiAlH_4$, DIBAL, K-selectride, L-selectride, $BH_3$, $NaBH_4$ and the combination thereof; the solvent is selected from ethers, alcohols, $H_2O$, alkanes, DMF, DMSO, urea and the combination thereof.

**9.** The process of claim 1, wherein the preparation of acetamide **5** from hydroxy(ether)amide **18** comprises :

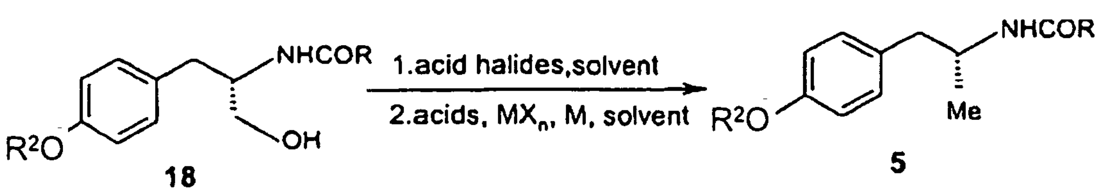


18 5

wherein an acid halide, a solvent, an organic acid, MXn and M are used; the acid halide is selected from the group of TsCl, MsCl, $SOCl_2$, $SO_2Cl_2$, $PCl_3$, $PCl_5$, $POCl_5$, oxalyl chloride and the combination thereof; the solvent is selected from THF, ketones, alkanes, ethers, DMF, DMSO, $CH_2Cl_2$, $CHCl_3$, $CCl_4$, urea and the combination thereof; the

organic acid is selected from oxalic acid $(COOH)_2$, RCOOH and the combination thereof, where R is H, alkyl, or aryl; the M is selected from Li, Na, K, Mg, Ca, Zn, Pt, Pd, Cu, Co, Mn, Fe, Ni, or Cd; the X is Cl, Br, I, or OAc; the n value is 1-3 based on the valence of the metal.

**10.** The process of claim 4, wherein the preparation of compound **21** from compound 20 comprises :

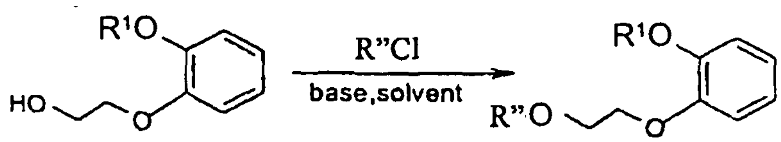


20 21

wherein a R"Cl, a base and a solvent are used; the R"Cl is selected from $MeC_6H_4SO_2Cl$ or $MeSO_2Cl$ and the combination thereof; the base is selected from $Et_3N$, $Me_3N$, pyridine and the combination thereof; the solvent is selected from $CH_2Cl_2$, acetone, THF, ether, ethyl acetate and the combination thereof.

**Patentansprüche**

**1.** Verfahren zur Herstellung von Tamsulosin und seinen Derivaten mit der folgenden Formel:

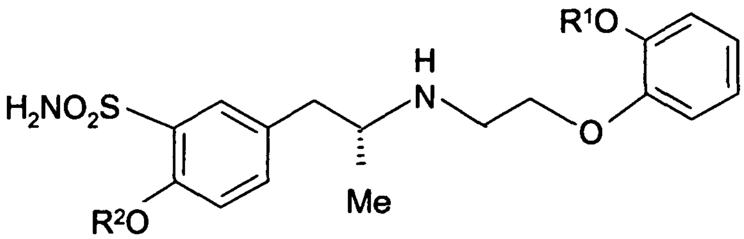

**1**

worin $R^1$ und $R^2$ $C_1$-$C_4$-Alkyl oder das Hydrochlorid desselben sowie verschiedene andere pharmazeutisch verwendete Salze repräsentieren, wobei das Verfahren den folgenden Schritt umfasst:

Umsetzen des Hydrochlorids von Sulfonamid 2 mit der Etherverbindung 21, um das Tamsulosin 1 zu erhalten:

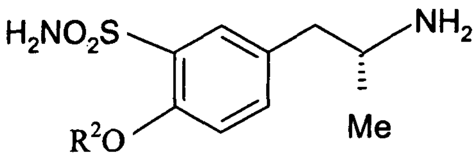

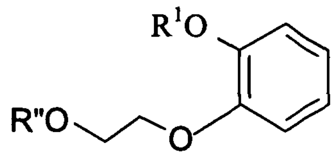

2 21

wobei in Formel 2 und 21 $R^1$ und $R^2$ der Definition in Form 1 entsprechen und R" $MeC_6H_4SO_2$ oder $MeSO_2$ repräsentiert, wobei bei dem Verfahren als zentrales Zwischenprodukt das folgende Acetamid 5 verwendet wird:

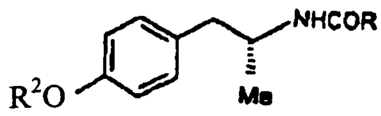

5

worin R Alkyl oder Aryl repräsentiert und $R^2$ der obigen Definition entspricht, wobei die nachfolgend dargestellten Zwischenprodukte für die Herstellung von Acetamid 5 verwendet werden:

(1) die (Phenol)amidosäure 15 mit der folgenden Formel:

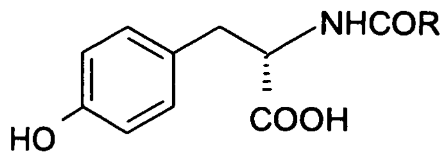

15

worin R Alkyl oder Aryl repräsentiert;
(2) der (Phenol)amidoester 16 mit der folgenden Formel:

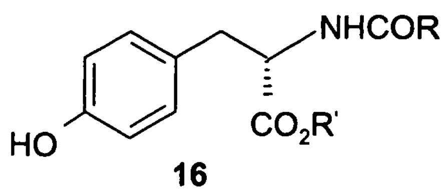

16

worin R und R' Alkyl oder Aryl repräsentieren;
(3) der (Ether)amidoester 17 mit der folgenden Formel:

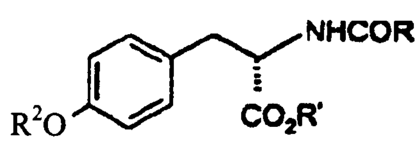

17

worin R und R' Alkyl oder Aryl repräsentieren; und $R^2$ der obigen Definition entspricht; und
(4) das Hydroxy(ether)amid 18 mit der folgenden Formel:

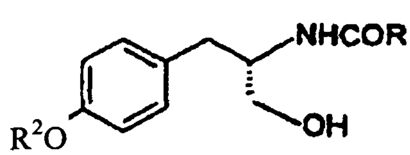

18

worin R Alkyl oder Aryl repräsentiert; und $R^2$ der obigen Definition entspricht.

**2.** Verfahren zur Herstellung von Tamsulosin nach Anspruch 1, wenn für Tamsulosin $R^1$ Ethyl ist und $R^2$ Methyl ist, $R^2$ in Formel 2 Me repräsentiert, wie durch 2A dargestellt, und das Hydrochlorid von 2A durch 2B dargestellt ist; in der Etherverbindung 21 steht $R^1$ für Et und R" steht für Ts, wie durch das (Ether)benzoxytosylat 21A dargestellt;

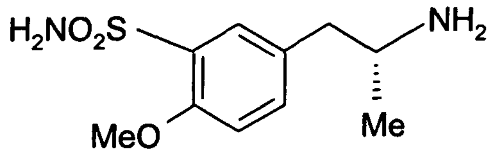

**2A**

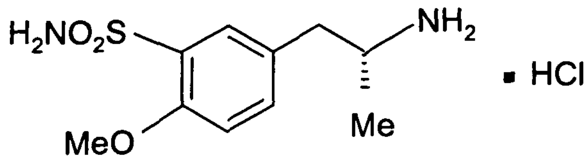

**2B**

und

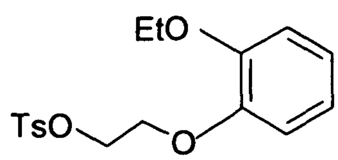

**21A**

**3.** Verfahren zur Herstellung von Tamsulosin nach Anspruch 2, wobei das Herstellungsverfahren für das Sulfonamidhydrochloridsalz 2B den folgenden Schritt umfasst:

Umwandeln von Acetamid 5A in (Sulfo)acetamid 6A, um dann Sulfonamidhydrochloridsalz 2B unter einer sauren Bedingung zu erhalten, wie aus den nachfolgend dargestellten Abläufen hervorgeht:

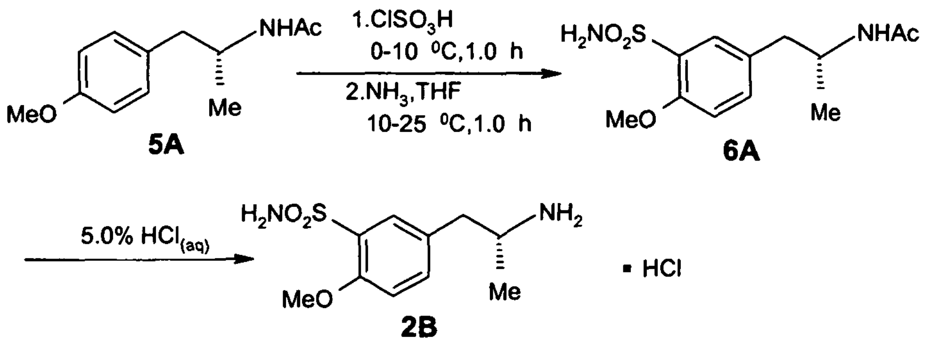


5A 6A 2B

4. Verfahren zur Herstellung von Tamsulosin nach Anspruch 1, wobei die Verbindung 21 hergestellt wird durch Umwandeln der nachfolgend dargestellten Verbindung 19 in die nachfolgend dargestellte Verbindung 20 und dann in die Verbindung 21:

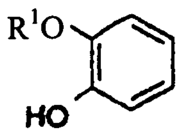

19

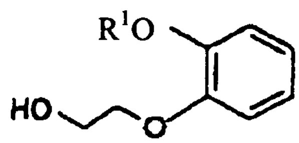

20

worin $R^1$ der Definition in Anspruch 1 entspricht.

5. Verfahren nach Anspruch 1, wobei die (Phenol)amidosäure 15 wie folgt aus dem Ausgangsmaterial L-Tyrosin 7 hergestellt wird:

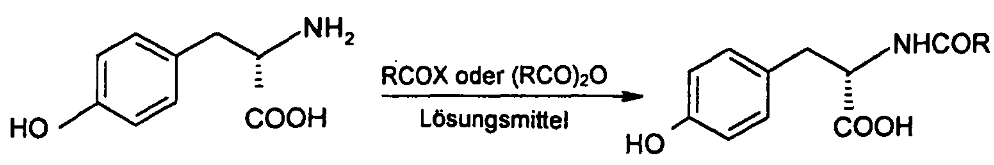


7 15

wobei ein Acylierungsmittel und ein Lösungsmittel verwendet werden; das Acylierungsmittel ist ausgewählt aus RCOX, $(RCO)_2O$ und der Kombination davon, worin R Alkyl oder Aryl ist; X ist ein Halogenid oder eine Abgangsgruppe; das Lösungsmittel ist ausgewählt aus Alkanen, Ethern, DMF, DMSO, Ketonen, Harnstoff und der Kombination davon.

6. Verfahren nach Anspruch 1, wobei die Herstellung von (Phenol)amidoester 16 aus (Phenol)amidosäure 15 Folgendes umfasst:

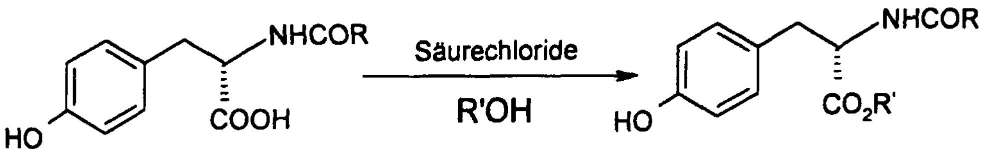


15 16

wobei ein Säurechlorid und ein R'OH verwendet werden; das Säurechlorid ist aus der Gruppe von $PCl_3$, $PCl_5$, $POCl_5$, $SOCl_2$, Oxalylchlorid und der Kombination davon ausgewählt; die Gruppen R und R' sind Alkyl oder Aryl.

**7.** Verfahren nach Anspruch 1, wobei die Herstellung von (Ether)amidoester 17 aus (Phenol)amidoester 16 Folgendes umfasst:

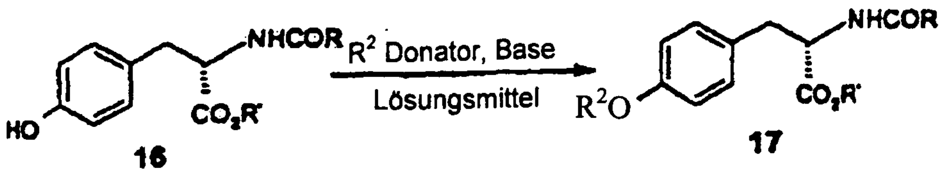


16 17

wobei ein Alkylierungsmittel, eine Base und ein Lösungsmittel verwendet werden; das Alkylierungsmittel ist aus $R^2{}_2SO_4$, $R^2I$, $R^2Br$ und der Kombination davon ausgewählt; die Base ist aus Aminen, Carbonaten, Hydrogencarbonaten, Amiden, Alkoxiden und der Kombination davon ausgewählt; das Lösungsmittel ist aus $H_2O$, Ketonen, Alkanen, Ethern, DMF, DMSO, Harnstoff und der Kombination davon ausgewählt.

**8.** Verfahren nach Anspruch 1, wobei die Herstellung von Hydroxy(ether)amid 18 aus (Ether)amidoester 17 Folgendes umfasst:

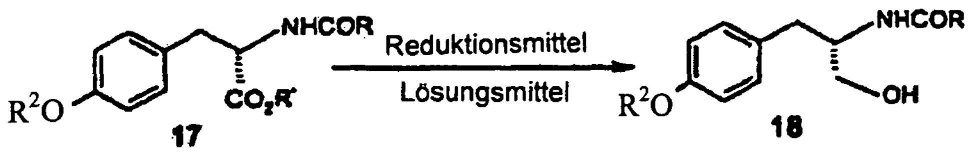


17 18

wobei ein Reduktionsmittel und ein Lösungsmittel verwendet werden; das Reduktionsmittel ist aus $LiAlH_4$, DIBAL, K-Selektrid, L-Selektrid, $BH_3$, $NaBH_4$ und der Kombination davon ausgewählt; das Lösungsmittel ist aus Ethern, Alkoholen, $H_2O$, Alkanen, DMF, DMSO, Harnstoff und der Kombination davon ausgewählt.

**9.** Verfahren nach Anspruch 1, wobei die Herstellung von Acetamid 5 aus Hydroxy(ether)amid 18 Folgendes umfasst:

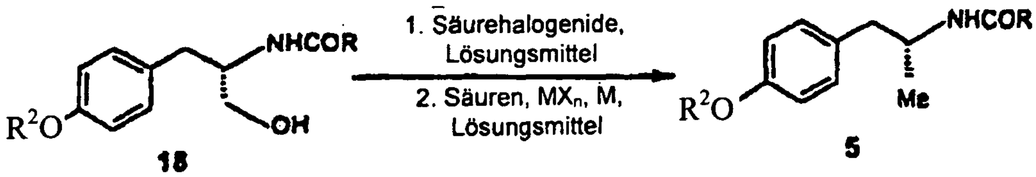


18 5

wobei ein Säurehalogenid, ein Lösungsmittel, eine organische Säure, $MX_n$ und M verwendet werden; das Säurehalogenid ist aus der Gruppe von TsCl, MsCl, $SOCl_2$, $SO_2Cl_2$, $PCl_3$, $PCl_5$, $POCl_5$, Oxalylchlorid und der Kombination davon ausgewählt; das Lösungsmittel ist aus THF, Ketonen, Alkanen, Ethern, DMF, DMSO, $CH_2Cl_2$, $CHCl_3$, $CCl_4$, Harnstoff und der Kombination davon ausgewählt;
die organische Säure ist aus Oxalsäure $(COOH)_2$, RCHOOH und der Kombination davon ausgewählt, wo R für H, Alkyl oder Aryl steht; M ist aus Li, Na, K, Mg, Ca, Zn, Pt, Pd, Cu, Co, Mn, Fe, Ni oder Cd ausgewählt; X ist Cl, Br, I oder OAc; der Wert n ist 1-3 bezogen auf die Valenz des Metalls.

**10.** Verfahren nach Anspruch 4, wobei die Herstellung der Verbindung 21 aus Verbindung 20 Folgendes umfasst:

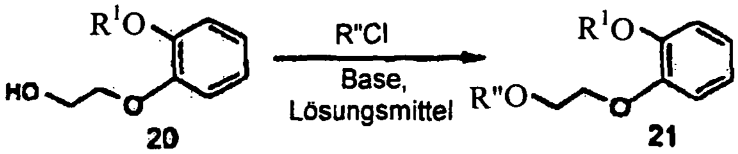


20 21

wobei R"Cl, eine Base und ein Lösungsmittel verwendet werden; das R"Cl ist aus $MeC_6H_4SO_2Cl$ oder $MeSO_2Cl$ und der Kombination davon ausgewählt; die Base ist aus $Et_3N$, $Me_3N$, Pyridin und der Kombination davon ausgewählt; das Lösungsmittel ist aus $CH_2Cl_2$, Aceton, THF, Ether, Ethylacetat und der Kombination davon ausgewählt.

## Revendications

**1.** Procédé de préparation de tamsulosine et de ses dérivés, de formule suivante :

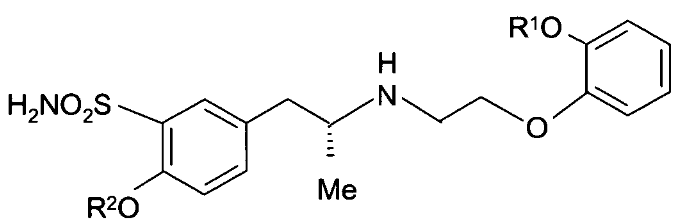

1

dans laquelle $R^1$ et $R^2$ représentent chacun un groupe alkyle en $C_{1-4}$, ou d'un chlorhydrate d'un tel composé, ou de divers autres sels d'un tel composé, utilisés en pharmacie, lequel procédé comporte :

le fait de faire réagir le chlorhydrate d'un sulfonamide de formule 2 avec un éther de formule 21, de manière à obtenir une tamsulosine de formule 1 :

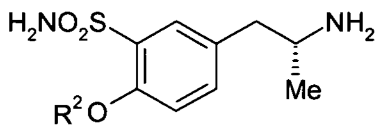

2

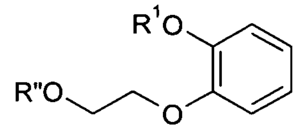

21

étant entendu que dans les formules 2 et 21, $R^1$ et $R^2$ ont les significations indiquées à propos de la formule 1, et R" représente un groupe symbolisé par $MeC_6H_4SO_2$ ou $MeSO_2$,
lequel procédé implique l'utilisation, en qualité d'intermédiaire clé, d'un acétamide de formule 5 suivante :

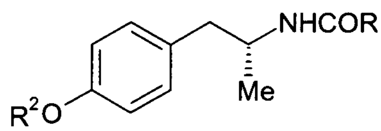

5

dans laquelle R représente un groupe alkyle ou aryle, et $R^2$ a la signification indiquée plus haut,
et pour lequel procédé on utilise les produits intermédiaires présentés ci-dessous pour préparer cet acétamide de formule 5 :

1) un phénol-amido-acide de formule 15 suivante :

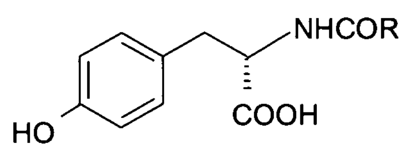

**15**

dans laquelle R représente un groupe alkyle ou aryle ;
2) un phénol-amido-ester de formule 16 suivante :

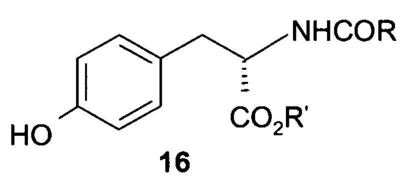

**16**

dans laquelle R et R' représentent chacun un groupe alkyle ou aryle ;
3) un éther-amido-ester de formule 17 suivante :

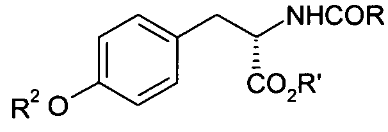

17

dans laquelle R et R' représentent chacun un groupe alkyle ou aryle et $R^2$ a la signification indiquée plus

haut ;

4) et un hydroxy-éther-amide de formule 18 suivante :

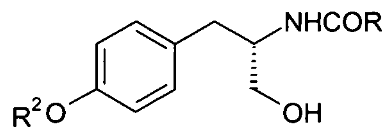

18

dans laquelle R représente un groupe alkyle ou aryle et $R^2$ a la signification indiquée plus haut.

**2.** Procédé de préparation de tamsulosine, conforme à la revendication 1, dans lequel :

- pour la tamsulosine 1, $R^1$ représente un groupe éthyle et $R^2$ représente un groupe méthyle ;
- dans la formule 2, $R^2$ représente un groupe méthyle, comme indiqué dans la formule 2A, et le chlorhydrate de ce composé de formule 2A est représenté par la formule 2B ;
- et dans l'éther de formule 21, $R^1$ représente un groupe éthyle et R" représente un groupe symbolisé par Ts, comme indiqué dans la formule 21A (éther-phénoxy-tosylate) :

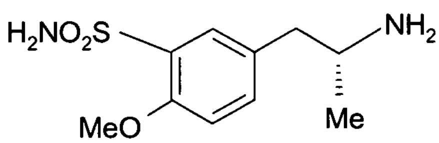

2A

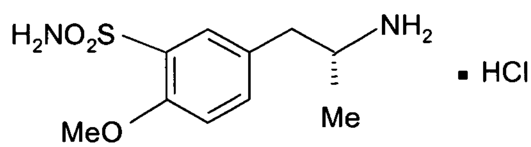

2B

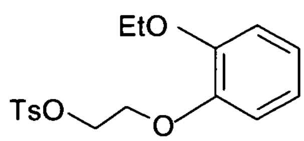

21A

.

**3.** Procédé de préparation de tamsulosine, conforme à la revendication 2, pour lequel le chlorhydrate de sulfonamide, sel de formule 2B, est préparé selon un procédé qui comporte le fait de convertir l'acétamide de formule 5A en le sulfonamide-acétamide de formule 6A, que l'on convertit ensuite, en milieu acide, en le chlorhydrate de sulfonamide de formule 2B, selon le schéma suivant :

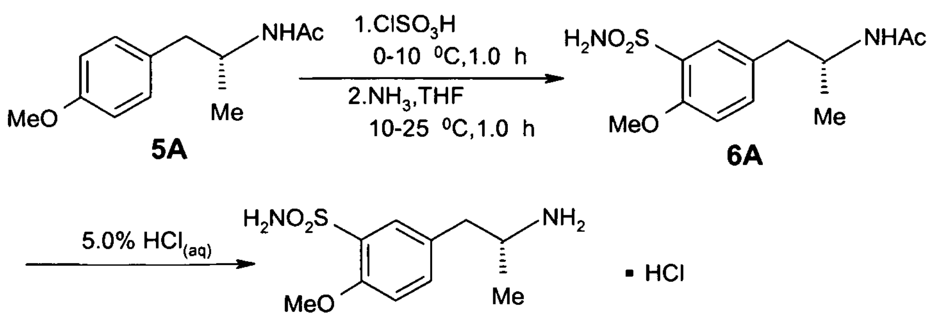


5A 6A 2B

.

**4.** Procédé de préparation de tamsulosine, conforme à la revendication 1, pour lequel on prépare le composé de formule 21 en convertissant un composé de formule 19 donnée ci-dessous en un composé de formule 20 donnée ci-dessous, puis celui-ci en un composé de formule 21 :

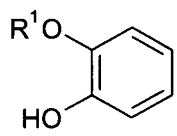

19

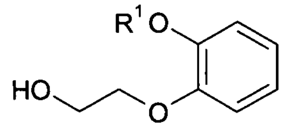

20

dans lesquelles formules $R^1$ a la signification indiquée dans la revendication 1.

**5.** Procédé conforme à la revendication 1, pour lequel on prépare un phénol-amido-acide de formule 15 à partir de L-tyrosine, produit de départ de formule 7, selon la réaction suivante :

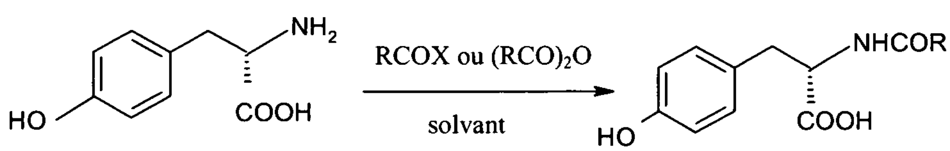


7

15

pour laquelle on utilise un agent d'acylation et un solvant, l'agent d'acylation étant choisi parmi les composés de formule RCOX ou $(RCO)_2O$ où R représente un groupe alkyle ou aryle et X représente un atome d'halogène ou un groupe partant, ainsi que les combinaisons de tels composés, et le solvant étant choisi parmi les alcanes, les éthers, le diméthyl-formamide, le diméthyl-sulfoxyde, les cétones et l'urée, ainsi que leurs combinaisons.

**6.** Procédé conforme à la revendication 1, pour lequel la préparation d'un phénol-amido-ester de formule 16, à partir d'un phénol-amido-acide de formule 15, comporte la réaction suivante :

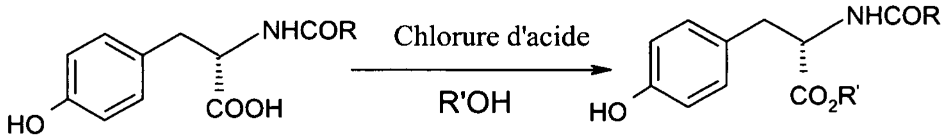


15

16

pour laquelle on utilise un chlorure d'acide et un composé de formule R'OH, le chlorure d'acide étant choisi parmi les composés de formules $PCl_3$, $PCl_5$, $POCl_5$ et $SOCl_2$ et le chlorure d'oxalyle, ainsi que les combinaisons de ces composés, et R et R' représentant chacun un groupe alkyle ou aryle.

**7.** Procédé conforme à la revendication 1, pour lequel la préparation d'un éther-amido-ester de formule 17, à partir d'un phénol-amido-ester de formule 16, comporte la réaction suivante :

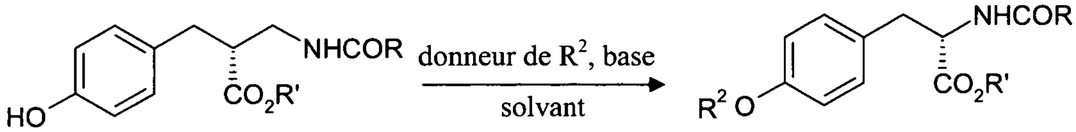


16

17

pour laquelle on utilise un agent d'alkylation, une base et un solvant, l'agent d'alkylation étant choisi parmi les composés de formule $R^2{}_2SO_4$, $R^2I$ ou $R^2Br$ ainsi que les combinaisons de tels composés, la base étant choisie parmi les amines, carbonates, hydrogénocarbonates, amides et alcoolates, ainsi que les combinaisons de tels composés, et le solvant étant choisi parmi l'eau, les cétones, les alcanes, les éthers, le diméthyl-formamide, le

diméthyl-sulfoxyde et l'urée, ainsi que leurs combinaisons.

**8.** Procédé conforme à la revendication 1, pour lequel la préparation d'un hydroxy-éther-amide de formule 18, à partir d'un éther-amido-ester de formule 17, comporte la réaction suivante :

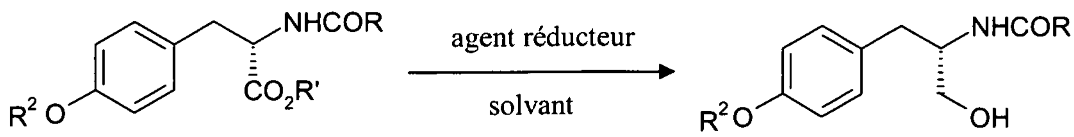


17 18

pour laquelle on utilise un agent réducteur et un solvant, l'agent réducteur étant choisi parmi les composés de formules $LiAlH_4$, $BH_3$ et $NaBH_4$, l'hydrure de di-isobutyl-aluminium (DIBAL), le L-selectride et le K-selectride, ainsi que les combinaisons de ces composés, et le solvant étant choisi parmi l'eau, les éthers, les alcools, les alcanes, le diméthyl-formamide, le diméthyl-sulfoxyde et l'urée, ainsi que leurs combinaisons.

**9.** Procédé conforme à la revendication 1, pour lequel la préparation d'un acétamide de formule 5, à partir d'un hydroxy-éther-amide de formule 18, comporte la réaction suivante :

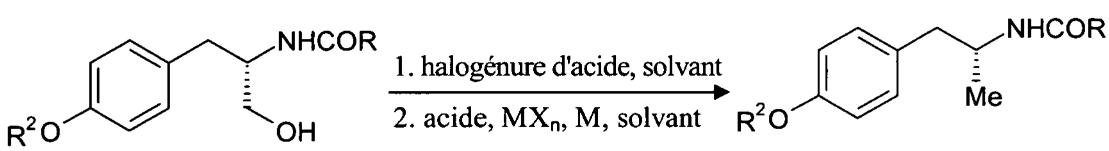


18 5

pour laquelle on utilise un halogénure d'acide, un solvant, un acide organique, un sel de formule $MX_n$ et un métal M, l'halogénure d'acide étant choisi parmi les composés de formules TsCl, MsCl, $SOCl_2$, $SO_2Cl_2$, $PCl_3$, $PCl_5$, $POCl_5$ et le chlorure d'oxalyle, ainsi que les combinaisons de ces composés, le solvant étant choisi parmi le tétrahydrofurane, les cétones, les alcanes, les éthers le diméthyl-formamide, le diméthyl-sulfoxyde, le dichlorométhane, le trichlorométhane, le tétrachlorure de carbone et l'urée, ainsi que leurs combinaisons, l'acide organique étant choisi parmi l'acide oxalique de formule $(COOH)_2$ et les composés de formule RCOOH où R représente un atome d'hydrogène ou un groupe alkyle ou aryle, ainsi que leurs combinaisons, le métal M étant choisi parmi les lithium, sodium, potassium, magnésium, calcium, zinc, platine, palladium, cuivre, cobalt, manganèse, fer, nickel et cadmium, X représentant un ion chlorure, bromure, iodure ou acétate, et l'indice n valant de 1 à 3 en fonction de la valence du métal.

**10.** Procédé conforme à la revendication 4, pour lequel la préparation d'un composé de formule 21, à partir d'un composé de formule 20, comporte la réaction suivante :

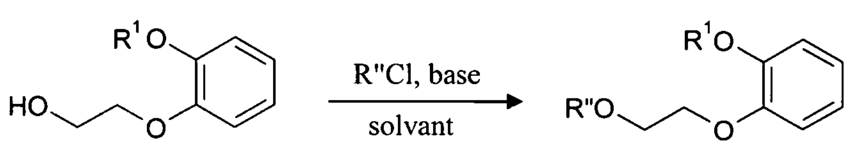


20 21

pour laquelle on utilise un composé de formule R"Cl, une base et un solvant, le composé de formule R"Cl étant choisi parmi les composés de formules $MeC_6H_4SO_2Cl$ et $MeSO_2Cl$, ainsi que leurs combinaisons, la base étant choisie parmi la triéthyl-amine, la triméthyl-amine et la pyridine, ainsi que leurs combinaisons, et le solvant étant choisi parmi le dichlorométhane, l'acétone, le tétrahydrofurane, un éther et l'acétate d'éthyle, ainsi que leurs combinaisons.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5447958 A
- US 4000197 A


### Non-patent literature cited in the description


- *J. Med. Chem.,* 1973, vol. 16, 480-483
- *Synth. Commun.,* 1998, vol. 28, 1935-1945